# EUROPEAN PATENT APPLICATION

(11) **EP 2 614 732 A1**
(43) Date of publication of application: **17.07.2013**
(21) Application number: 13151107.3
(22) Date of filing: 14.01.2013
(51) Int. Cl.: A24F 47/00

(54) **Cartridge of electric cigarette for preventing leakage**

(30) Priority: 13.01.2012 KR 20120004137
(71) Applicant: Kim, Han Ki, Gyeonggi-do 463-923 (KR)
(72) Inventor: Lee, Yong Yin, 134-858 Seoul (KR)
(74) Representative: Hernández, Yorck

(57) **Abstract**

The present invention relates to a cartridge of electric cigarette for preventing leakage. The cartridge may comprise a storing container 11 to store liquid therein; an extending tube 12 to have a guide tube 121 installed within the storing container 11 and extending in a hollow cylindrical shape, a lower fixing plate 122 coupled to the lower part of the guide tube 121 and secured to the lower part of the storing container 11 and a fixing gap 123 formed at the upper part of the guide tube 121; a guide lid 16 coupled to the upper part of the extending tube 12 and introducing evaporated component; an evaporating unit 17 fixed at the fixing gap 123; and a mouth piece 13 communicating with the guide lid 16 and coupled to the upper part of the storing container 11.

## Description

### BACKGROUND OF THE INVENTION

### 1. Field of the Invention

The present invention relates to a cartridge of an electric cigarette for preventing leakage, in particular a cartridge of an electric cigarette for preventing leakage in which an evaporating unit is displaced at the upper part of a storage container for preventing some solution from leaking and enhancing evaporating property.

### 2. Description of the Related Art

An electric cigarette means a device by which some solution such as nicotine solution and aid solution for stopping smoking can be evaporated into gas by means of heat of electric resistor to be inhaled into human body. In general, the electric cigarette may comprise a battery assembly, a supplying assembly, an evaporating assembly and an inhaling assembly. The battery assembly may comprise a battery such as a rechargeable secondary battery and can supply power to evaporate the solution in the supplying assembly. The supplying assembly may store nicotine solution or aid solution for stopping smoking to provide constant amount with the evaporating assembly on inhaling. The evaporating assembly may evaporate the provided solution into gas with the power provided from the battery assembly. And the inhaling assembly has the function that the gas from the evaporating assembly may be directed to a mouth for being inhaled.

A relevant electric cigarette was described in Korean Utility Model Publication No. 20-2011-0010862. The above invention describes a cartridge integrated into an electric cigarette in a single body and the cartridge comprises an essence casing to the upper part of which a cigarette body is connected and to the lower part of which a inhaling tube part is connected, and which contains essence solution in the form of cylinder; a lower part cap which is secured in the lower part of the essence casing and connects the essence casing to the cigarette body in a removable manner; an air inlet which is displaced through the lower and extends the inner upper part of the essence casing; an atomizing tube body coupled to the upper part of the air inlet in the form of cylinder, having an air inlet coupling part connected to the air inlet in the lower direction and two safe displacing grooves in the upper part of a main wall to the opposite each other; a wick which is displaced on a first air passage by being inserted into the safe displacing grooves and the end part of at least one extends into the essence casing; a heating coil which warps the wick for heating; a packing part which is inserted through the upper part of the essence casing for preventing leakage and which has a run-through hole in the center part for providing a second air passage on the extending line of the first air passage; and a power supplying means to provide the heating coil with the power of the battery displaced in the cigarette body.

As another prior art of the electric cigarette, a cartridge integrated into an atomizer for an electric cigarette is described in Korea Patent Registration No. 1081481. The above invention describes an cartridge comprising a solution tank which forms an essence storing volume and has a smoking outlet hole in an open lower part; a body which is displaced into the essence storing volume and connected to the lower part of a smoking flow tube to contain a smoking flow hole communicating with the smoking outlet hole and the essence tank; and a heating element displaced in the inner part of the body for heating the essence.

Referring to the above-mentioned inventions, there is disadvantage that the evaporating element is displaced in a lower part of the storing container and the wick has to extend to the solution storing container even though the evaporating element is displaced in the upper part of the storing container. If the evaporating assembly is displaced in the lower part of the liquid storing container, then a guide passage has to be formed for the evaporated component, and if the wick is extended, then some contaminating material occurs in the liquid storing container. And also, if wiring for transmitting electric power to the heater is in the liquid storing container, the total structure of the electric cigarette may become complicate.

The present invention suggests a cartridge having different structure from the prior art to solve disadvantage of the prior art.

### SUMMARY OF THE INVENTION

A purpose of the present invention is to provide a cartridge of an electric cigarette to prevent some liquid from leaking and to have enhanced evaporating property.

According to a first embodiment of the present invention, a cartridge of an electric cigarette may comprise a storing container to store liquid therein; an extending tube to have a guide tube to be displaced within the storing container, a lower fixing plate coupled to the lower part of the guide tube and a fixing gap formed at the upper part of the guide tube; a guide lid to be coupled to the upper part of the extending tube and to guide evaporated component; an evaporating unit to be fixed into the fixing gap; and a mouth piece to communicate with the guide lid and coupled to the upper part of the storing container, wherein the guide lid prevents the liquid of the storing container from inflowing into the upper part of the extending tube and the liquid of the storing container is provided to the evaporating unit through the fixing gap.

According to a second embodiment of the present invention, the guide lid may comprise a hollow type of a sealing element coupled to the upper part of the extending tube and to have an outlet at one end, and an protruding tube to be placed within the sealing element and to allow for flow of evaporated component by being inserted within the outlet and be protruded out of the extending tube.

According to a third embodiment of the present invention, the evaporating unit may comprise a heating element to be coupled to a positive terminal and a negative terminal and a wiring for the positive terminal and the negative terminal may be placed within the extending tube.

The cartridge according to the present invention has advantage that the leakage of the liquid can be prevented to avoid an erroneous operation of the electric cigarette due to humidity. And also, the cartridge according to the present invention may have enhanced sanitation by being no wiring within the storing container and separating the evaporating unit from the part of liquid-storing unit. Furthermore, the cartridge according to the present invention has advantage that the components may be formed in an integrated shape to make production simple.

### BRIEF DESCRIPTION OF THE DRAWINGS

The above and other aspects, features and advantages of certain exemplary embodiments of the present invention will be more apparent from the following detailed description taken in conjunction with the accompanying drawings, in which:
FIG.1A and 1B is an exploded perspective view and a sectional view of an evaporating configuration, respectively, according to an embodiment of the present invention.
FIG.2 is an evaporating configuration according to other embodiment of the present invention.

### DETAILED DESCRIPTION OF EXEMPLARY EMBODIMENTS

Exemplary embodiments of the present invention will be described herein below with reference to the accompanying drawings. In the following description, well-known functions or constructions are not described in detail since they would obscure the invention in unnecessary detail. Also, the terms used herein are defined according to the functions of the present invention. Thus, the terms may vary depending on user's or operator's intension and usage. That is, the terms used herein must be understood based on the descriptions made herein.

An electric cigarette means an electric device to evaporate liquid with electric power, and liquid may comprise any kind of nicotine solution and aid solution for stopping smoking. And, also, liquid may comprise any solution inhaled into the human body by being evaporated from solution, and therefore the present invention will not be limited to the kind of liquid.

The present invention will be described in detail below.

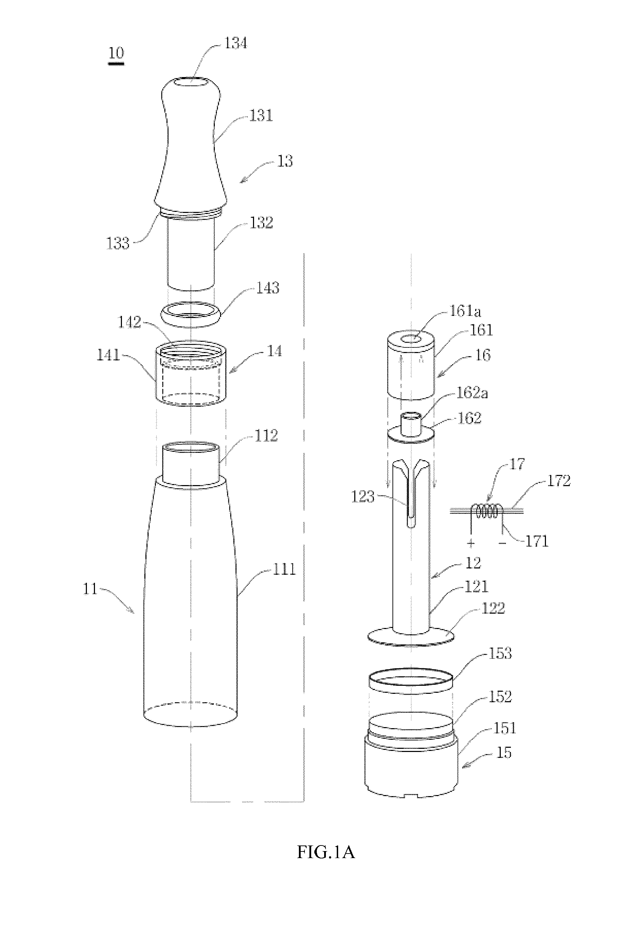

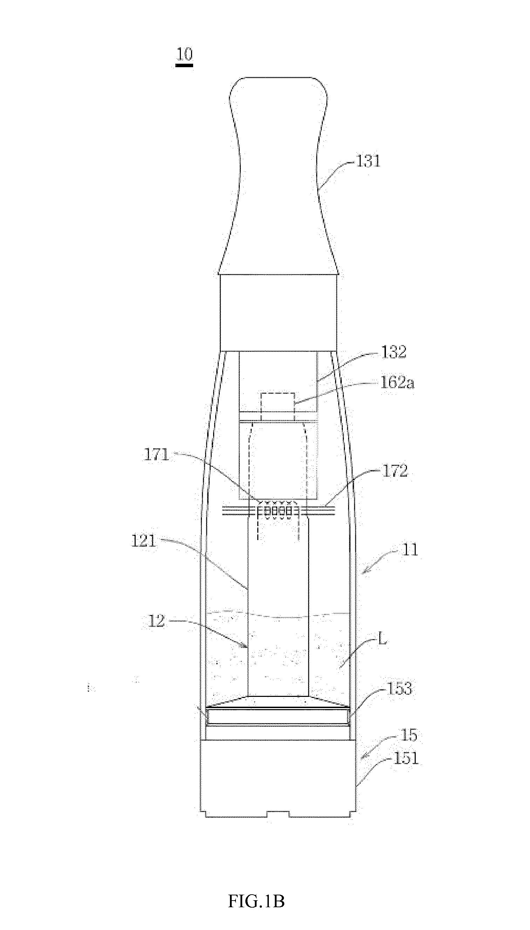

FIG.1A and 1B is an exploded perspective view and a sectional view of an evaporating configuration according to an embodiment of the present invention.

Referring to FIG.1A and FIG. 1B, a cartridge 10 of an electric cigarette may comprise a storing container 11 to store liquid therein; an extending tube 12 to have a guide tube 121 to be displaced within the storing container 11, a lower fixing plate 122 coupled to the lower part of the guide tube 121 and a fixing gap 123 formed at the upper part of the guide tube 121; a guide lid 16 to be coupled to the upper part of the extending tube 12 and to guide evaporated component; a evaporating unit 17 to be fixed into the fixing gap 123; and a mouth piece 13 to communicate with the guide lid 16 and coupled to the upper part of the storing container 11, wherein the guide lid 16 prevents the liquid of the storing container 11 from inflowing into the upper part of the extending tube 16 and the liquid of the storing container 11 is provided to the evaporating unit 17 through the fixing gap 123.

The cartridge 10, in general, may distinguish from a battery assembly or a mouth piece and may comprise a storing assembly and an evaporating assembly.

According to the present invention, the cartridge 10 may comprise at least an evaporating unit and a liquid supplying unit. But the cartridge 10 according to the present invention may comprise a mouth piece in some embodiments.

The storing container 11 may have a hollow cylindrical shape and the upper side and lower side may be opened. But the present invention is not limited the shape of the storing container 11 and the storing container 11 may have any shape to store some liquid and to displace the extending tube 12.

The extending tube 12 may be installed within the storing container 11 and arranged in a hollow type of tube extending from the lower part to the upper part of the storing container 11. As described in following, wiring for providing electric power with the heat element 17 may be displaced within the extending tube 12. The lower fixing plate 122 coupled to the lower part of the extending tube 12 may form a bottom of the storing container 11 and may support the extending tube 12. The lower fixing plate 122 may be secured to the lower part of the extending tube 12 in various ways.

A connecting body 15 may be coupled to the lower part of the storing container 11. The connecting body 15 may seal the lower part of the storing container together with the lower fixing plate 122 and may couple the cartridge to a battery assembly (not shown). The connecting body 15 may be made various forms and be made in a single shape or may comprise a plural of components. For example, the connecting body 15 may comprise a lower body 151, and an upper connecting body 152 which is formed above the lower body 151 for supporting the lower fixing plate 122. And also, the connecting body 15 may have an air passage for supplying the outer air into the evaporating unit 17 through the extending tube 12. A sealing means 153 may be arranged between the lower fixing plate 122 and the connecting body 15. The sealing means 153 has the function that the leakage of liquid below the lower fixing plate 122 , and the sealing means 153 may be a sealing ring made from silicone, rubber or the like. The lower body 151 and the upper connecting body 152 seals the lower part of the storing container 11 and may have a proper structure for installing the sealing means 153. Hence, the present invention is not limited to the above-mentioned examples.

The lower fixing plate 122 may be integrated into the upper part of the connecting body 15. If the lower fixing plate 122 is fixed at the upper part of the connecting body 15, a space for inserting the sealing means 153 between the upper part of the connecting body 15 and the lower fixed plate 122 may be formed. The lower fixing plate 122 and the connecting body 15 may have various structures for installing the sealing means153, and the present invention is not limited to the example.

The guide tube 121 may extend from the lower fixing plate 122 to the upward direction and may be in a hollow type of tube. The outer air from the connecting body 15 may be introduced into the guide tube 121 through a penetrating hole (not shown) formed at the lower fixing plate 122. The outer air introduced into the guide tube 121 may be supplied to the mouth piece 13 together with the evaporated component generated at the evaporating unit 17. The evaporating unit 17 and a fixing gap 123 for supplying liquid to the evaporating unit 17 may be formed at the upper part of the guide tube 121. As shown in FIG.1A and FIG.1B, the fixing gap 123 may be form in a slit structure extending from the upper end of the guide tube 121 to the downward by some length. If the evaporating unit 17 is formed as a plural of fiber strains and electric wiring resistor, the fixing gap 123 may have a proper structure for fixing both end parts of the plural of fiber strains. And also, the fixing gap 123 may be a pair of gaps opposite each other, but the present invention is not limited to the particular form.

The guide lid 16 may be coupled to the upper part of the guide tube 121. The guide tube 15 may seal the upper part of the guide tube 121 and introduce the outer air and the evaporated component into the mouth piece 13. Specifically, the guide lid 16 may comprise a hollow cylindrical type of sealing member 161 which is coupled to the upper part of the extending tube 12 and an outlet 161a is formed on one side with other side open, an protruding tube 162a which is fixed in a removable manner within the sealing member 161 and is inserted into the outlet 161a to protrude out of the extending tube 121 for introducing the outer air and the evaporating component, and an attached plate 162 which is coupled to the lower part of the protruding tube 162a and secures the sealing member 161 within the guide lid 16. The sealing member 151 may be made from flexible material such as silicone or rubber and the attached plate 162 may be integrated into the protruding tube 162a to form a single shape. On the other hand, the attached plate 162 or the protruding tube 162a may be made from rigid material such as metals, and hence the protruding tube 162a can be inserted into the outlet 161a of the sealing member 161 with flexibility.

The evaporating unit 17 may comprise a heating element 171, for example Ni-Cr wire, for generating heat with electric power and a liquid transferring means 172 for evaporating the liquid supplied from the storing container with the heat generated from the heating element 171. The liquid transferring means 172 may be form as a plural of fiber strains and the evaporating unit 17 has a structure that the Ni-Cr wire winds the plural of fiber strains. If the liquid transferring means 172 is formed from the plural of fiber strains, both end parts of the liquid transferring means 172 may extend above the outer surface of the guide tube 121 through the fixing gap 123. In such case, the liquid stored in the storing container 11 may be transferred to the heating member 171 via the liquid transferring means 172 using capillary phenomenon. The evaporating unit 17 may be constructed in various ways and hence, the present invention is not limited to the depicted example.

The storing container 11 may comprise a storing body 111 and a connecting part 112 which is formed in the upper part of the storing body 111. And a fixing member 14 may be coupled to the connecting part 112. The fixing member 14 may have a proper configuration that one end is coupled to the connecting part 112 while the other end is coupled to the mouth piece 13. For example, the fixing member 14 may comprise a hollow type of coupling part 141 and a threading part 142 which allows for the threaded connection of the mouth piece 13. The storing container 11 may have the connecting part 112 optionally, and also the fixing member 14 may be optional component according to the cartridge of the present invention. The mouth piece 13 may be connected to the upper part of the storing container 11 directly.

The mouth piece 13 may have any proper configuration that the evaporated component and the outer air from the guide tube 121 and the guide rid 16 can be introduced to the out of the cartridge. For example, the mouth piece 13 may comprise an introducing tube 132 which extends into the storing container 11 to contact the upper of the guide lid 16, an inhaling member 131 which introduces the evaporating component from the introducing tube 131, an inhaling hole 134 which is formed at the end part of the inhaling member 131 and a connecting thread part 133 which is threaded with the threading part 142. A gas seal packing 143 may be installed between the fixing member 14 and the mouth piece 13 for preventing the evaporating component from leaking, if necessary.

The mouth piece 13, the gas seal packing 143 or the fixing member 14 may has any configuration known in the art, and the present invention is not limited to any kinds of these.

Other embodiment according to the present invention will be described in following.

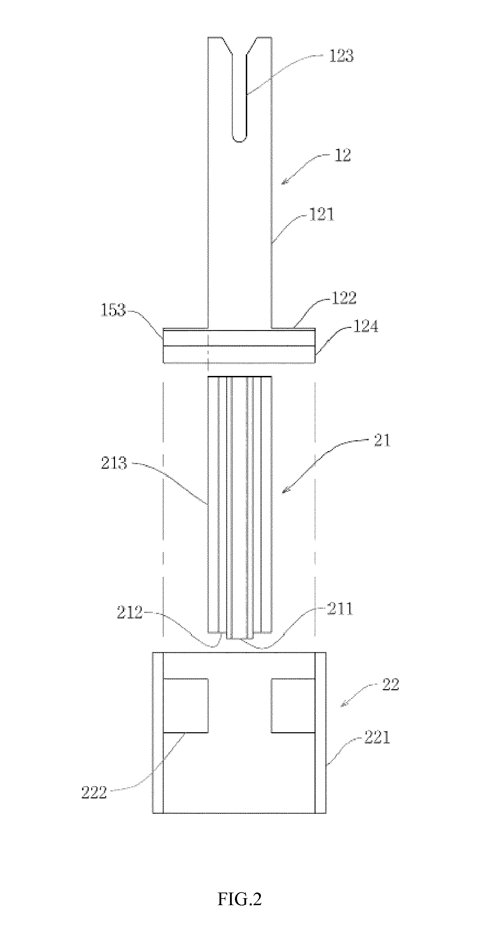

FIG.2 is an evaporating configuration according to other embodiment of the present invention.

In regard of the drawings, the like numerals in different drawing indicate a component having the same or similar function, and hence any component having the like numerals may not described repeatedly for brevity and simplicity.

Referring to FIG. 2, the lower fixing plate 122 may extend in the downward and be coupled an extending part 124 in which the sealing means 153 can be inserted. The lower fixing plate 122 may be coupled to the extending part 124 or be integrated into the extending part 124 to form in a single body.

A coupling member 22 may be secured at the lower part of the storing container (referring to FIG.1A) in order to seal the lower part of the storing container and connect the storing container to a battery assembly. The coupling member 22 has a similar to that of the connecting body 15.

The coupling member 22 may comprise a hollow cylindrical type of outer housing 221 and a joint fixing member 222 which is displaced within the outer housing 221. The guide body 21 may be fixed at the joint fixing member 222.

The guide body 21 may comprise an air passage 211 to introduce the outer air, an insulating member 212 to surround the air passage 211, and a fixing housing 213 to surround the insulating member 212 and to secure the guide body 21 to the joint fixing member 222. The insulating member 212 may insulate between the guide body 21 and the extending tube 12 electrically as well between the guide body 21 and the coupling member 22. According to the present invention, the guide body 21, the joint fixing member 222 and the outer housing 221 may be made from conductive material. In such case, one electrode of the heating element 171 described in FIG. 1A may be connected to the guide tube 121, while the other electrode may be connected to the air passage 211. And the air passage 211 and the outer housing 221 may be connected to the terminals of a battery each other in proper way. The above configuration that the guide tube 121 and the outer house 221 is used as wiring between the battery and the heating element 171 may have advantage that some wiring within the storing container is not required to prevent liquid from being contaminated owing to any wiring.

The guide body 21 may be integrated into the lower fixing plate 122 or the extending part, if necessary. In case of the example described in FIG.2, the upper part of the guide body 21 may be fixed at the extending part 124 and the lower part may be fixed at the joint fixing member 222. And the lower fixing plate 122 may be coupled to the upper part of the coupling member 22.

The wiring among the guide body 21, the coupling member 22, the heating element 171 and the battery may be formed according to the known method, and the present invention is not limited to any specific configuration.

While the present invention has been particularly shown and described with reference to exemplary embodiments thereof, it will be understood by those skilled in the art that various changes in form and details may be made therein without departing from the spirit and scope of the present invention as defined by the appended claims.

### Description of the numerals

11: Storing container 12: Extending tube
13: Mouth piece 14: Fixing member
15: Coupling body 16: Guide lid
17: Evaporating unit 21: Guide body
22: Coupling member
111: Storing Body 112: Connecting part
121: Guide tube 122: Lower fixing plate
123: Fixing gap 124: Extending Part
131: Inhaling member 132: Introducing tube
133: Connecting thread part 134: Inhaling hole
141: Coupling part 142: Threading part
143: Gas Sealing Packing
151: Lower Body 152: Upper connecting Body
153: Sealing means 161a: Outlet
161: Sealing member 162a: Protruding tube
171: liquid transferring means
211: Air passage 212: Insulating member
221: Outer housing 222: Joint fixing member

## Claims

1. A cartridge for an electric cigarette, comprising:
a storing container 11 to store liquid therein;
an extending tube 12 to have a guide tube 121 installed within the storing container 11 and extending in a hollow cylindrical shape, a lower fixing plate 122 coupled to the lower part of the guide tube 121 and secured to the lower part of the storing container 11 and a fixing gap 123 formed at the upper part of the guide tube 121;
a guide lid 16 coupled to the upper part of the extending tube 12 and introducing evaporated component;
an evaporating unit 17 fixed at the fixing gap 123; and
a mouth piece 13 communicating with the guide lid 16 and coupled to the upper part of the storing container 11,
wherein the guide lid 16 prevents the liquid stored in the storing container 11 from inflowing into the upper part of the extending tube 16, the liquid of the storing container 11 can be supplied to the evaporating unit through the fixing gap 123, and the guide lid 16 includes a hollow type of an sealing member 161 coupled to the upper part of the extending tube 12 with an outlet 161a on the one side thereof, and a protruding tube 162a coupled to the inner part of the sealing member 161, inserted into the outlet 161a to protrude out of the extending tube 12 for the evaporating component.

2. The cartridge according to claim 1, wherein the evaporating unit 17 comprises a heating element 171 connected to a positive terminal and a negative terminal of a battery and wiring between the terminals and the battery is displaced within the extending tube 12.
